# EUROPEAN PATENT SPECIFICATION

(11) **EP 0 494 417 B1**
(45) Date of publication and mention of the grant of the patent: **17.04.1996**
(21) Application number: 91121769.3
(22) Date of filing: 19.12.1991
(51) Int. Cl.: C09H 7/00, A23L 1/0562, A61K 9/40

(54) **Process for crosslinking gelatin and for incorporation a material therein**
Verfahren zur Vernetzen von Gelatine und Aufnahme eines Materials
Procédé de réticulation de gélatine et incorporation d'un matériau

(30) Priority: 10.01.1991 US 639468; 10.01.1991 US 639646
(43) Date of publication of application: 15.07.1992
(73) Proprietor: BASF CORPORATION, Parsippany, New Jersey 07054 (US)
(72) Inventor: Chaundy, Frederick Kenneth, Grosse Ile, Michigan 48138 (US); Bower, David Kenneth, Wyandotte, Michigan 48192 (US); Kilbride, Terence Kevin, Jr., Bloomfield Hills, Michigan 48302 (US)
(74) Representative: Michaelis, Wolfgang, Dr.

(56) References cited:
- EP-A- 0 345 885
- US-A- 2 196 146
- US-A- 4 670 247
- CENTRAL PATENTS INDEX, BASIC ABSTRACTS JOURNAL Week 7346, Derwent Publications Ltd., London, GB; AN 70473U & JP-B-48 037 816

## Description

The invention relates to crosslinking gelatin. More specifically, the invention pertains to incorporating materials in the crosslinked gelatin.

The process of the present invention requires the use of a selected sugar (or sugars), a selected salt (or salts) and at least a minimal amount of water, and, if desired, at least one additional ingredient which is to be encapsulated. This particular combination of ingredients permits the crosslinking to be performed at temperatures previously inoperable to obtain the desired degree of crosslinking with ingredients that are safe for both food and animal feed applications. This "low temperature advantage" is very important because it permits crosslinking to be performed in the presence of the many heat sensitive materials (which of course have been present during the crosslinking process) without substantial thermal degradation thereof.

Thus the present invention is applicable to a wide range of arts, such as food sciences, photographic sciences, pharmaceuticals, etc, i.e. wherever gelatin crosslinking is utilized in combination with any additional chemical species which may be thermally sensitive, as well as in all situations in which it is advantageous to conserve energy. The invention is particularly useful in the vitamin arts, especially vitamin A, which undergoes thermal degradation at the temperatures previously required for obtaining substantial gelatin crosslinking with commonly used food ingredients. Thus the process of the present invention is particularly valuable for crosslinking gelatin in the presence of temperature-sensitive ingredients, such as vitamins, without substantial thermal degradation of the temperature sensitive ingredient as well as without requiring the use of a toxic crosslinking agent such as formaldehyde. As stated above, the advantages of the process of the present invent ion are especially important with respect to producing gelatin-encapsulated vitamin A products. Gelatin and sugar are commonly used food and feed ingredients and the salt may be one which is "generally recognized as safe" (i.e. "GRAS"). Thus one is able to use the process of the present invention to obtain a vitamin A encapsulated in a crosslinked gelatin wherein the process is carried out at a temperature at which there is no substantial thermal degradation of the vitamin A.

U.S. 2,196,146 describes subject matter which is related to the present invention. The '146 patent relates to improved food products and process for making the same, and particularly to food products containing sugar and acid, such as those commonly known as gelatin desserts that generally are marketed in the form of a dry powder and usually comprise gelatin. Among the acids mentioned in the '146 patent are "fruit acids" (such as tartaric acid), and to the additional use of salts of organic acids such as acetates. The '146 patent repeatedly refers to the "setting" of the gelatin.

In contrast to the '146 patent, the present invention requires that the gelatin be "crosslinked". Crosslinking differs from "setting" in that crosslinking is irreversible whereas setting (also known as "gelation") is reversible. The process of setting involves the transformation of a solution to a gel. The addition of heat to the gel can then be used to melt the gel so that a solution is formed. In stark contrast, crosslinking involves an irreversible chemical reaction, in that the addition of heat to the crosslinked protein will not result in transforming the crosslinked protein into a solution of the protein.

Another related patent is U.S. 4,500,453 relating to crosslinked collagen-derived protein compositions having improved Bloom gel strength and increased viscosity. Furthermore, the '453 patent relates to a process for crosslinking the protein with an aluminum salt of acetic acid. The '453 patent requires the use of an aluminum salt, as can be seen from the comparative example therein (Example 2) which shows the substitution of a sodium salt for the aluminum salt renders the process of the '453 patent inoperable.

In contrast, the present invention differs from the '453 patent in that the process of the present invention requires the use of at least one of a group of sugars. If one were to alter the process of the present invention by carrying it out in the absence of at least one of these sugars, the required degree of crosslinking will not result. Note Example 2 herein, which proves that upon using, for example, calcium acetate as the salt, the process of the present invention is inoperable (no crosslinking occurs) in the absence of at least one of the group of sugars.

U.S. 4,670,247 refers to a process for the preparation of fat-soluble vitamin active beadlet compositions which exhibit stability when exposed to the feed pelleting process. The process includes forming an aqueous emulsion of a fat-soluble vitamin-active material, gelatin, and a sugar, converting the emulsion to dry particulate form containing the non-aqueous constituents of the emulsion, and heat treating the resulting product to form water insoluble beadlets.

In contrast to the '247 patent, the process of the present invention requires the use of at least one of a group of salts. The '247 nowhere refers to either mandatory use of (or even optional use of) any salt whatsoever. Note Example 2 herein, which prove that at a preferred temperature (75°C for the product ion of encapsulated vitamin A) for the process of the present invention, inoperability results in the absence of at least one of the group of salts specified.

The present invent ion relates to both a process for making a crosslinked gelatin for incorporating a material therein, as well as to a crosslinked gelatinous product containing, if desired, that material. In general, the process of the present invention is carried out by first making an aqueous composition which comprises:
(1) gelatin
(2) a sugar which is at least one member selected from the group consisting of fructose, and glucose,
(3) a salt which is at least one member selected from the group consisting of water-soluble salts of carboxylic acids, sodium carbonate, potassium carbonate, calcium sulfate, and calcium phosphate,
(4) water, and
(5) optionally an additional ingredient.

The aqueous composition is then heated while the moisture content of the composition is maintained at a level of at least about 3 weight percent. Both the making of the composition (i.e. the selection and proportioning of the ingredients) as well as the heating of the composition, are to be carried out so that the gelatin is crosslinked to a degree at which it is water insoluble upon being placed in boiling water (i.e. water at 100°C) for at least three minutes.

In general, the product of the present invention comprises:
A. gelatin wherein the gelatin is crosslinked to a degree at which it is substantially water insoluble upon being placed in water at 100°C for at least 3 minutes;
B. a sugar, wherein the sugar is at least one member selected from the group consisting of fructose, and glucose;
C. a salt which is at least one member selected from the group consisting of water-soluble salts of carboxylic acids, sodium carbonate, potassium carbonate, calcium sulfate, and calcium phosphate;
D. water;
E. an additional ingredient.

It is an object of the present invention to provide a process for crosslinking gelatin, preferably in the presence of an additional ingredient, with the crosslinking reaction utilizing a sugar and a GRAS salt.

It is a further object of the present invention to crosslink gelatin to a degree of insolubility in boiling water for at least three minutes, without elevating the gelatin to a temperature which causes the degradation of an additional ingredient which is present during the crosslinking reaction.

It is a further object of the present invention to provide a process for crosslinking gelatin to a degree of insolubility in boiling water for at least three minutes, the crosslinking taking place at a temperature below 90°C, the crosslinking taking place preferably in the presence of a fat-soluble vitamin product.

It is a further object of the present invention to provide a process for crosslinking gelatin utilizing a GRAS salt, a sugar, and water, while heating the gelatin to a temperature of from 55°C to 85°C, wherein the heating is maintained for a period of from 2 hours to 24 hours, in the presence of a fat soluble vitamin.

It is a further object of the present invention to provide a crosslinked vitamin product comprising gelatin which has been crosslinked to a degree that it is insoluble in boiling water at 100°C for at least 3 minutes, a sugar, a GRAS salt, water, and a fat soluble vitamin which is substantially encapsulated within the crosslinked gelatin.

The phrases "crosslinked gelatin", as commonly used, pertain to a wide spectrum of products having widely differing degrees of crosslinking. The effects of the crosslinking range from a mere increase in the viscosity of the product, to the formation of a very rigid and brittle product which is of course completely insoluble in water. In the field of encapsulating vitamins, pharmaceuticals, food additives, etc, it is desirable to crosslink gelatin, to a degree that the resulting crosslinked matrix is substantially insoluble in boiling water for at least three minutes. If less than this amount of crosslinking is achieved, the encapsulated product will frequently escape during subsequent processing (ego pelleting or extrusion), resulting in undesirable degradation, etc of the encapsulated product.

Crosslinking is to be distinguished from gelation which is reversible by heating in that gelation is the result of hydrogen bonding between individual polymer molecules to form an infinite, 3-dimensional network whereas crosslinking is the result of a chemical reaction between polymer molecules. If polymer molecules are permitted to crosslink to a certain degree, the result is that the crosslinking reaction is irreversible.

Heating will generally not reverse a crosslinking reaction. However, reversal of a crosslinking reaction may occur, at least to some degree, if the crosslinking reaction is so slight that the crosslinked bonds between polymer molecules are not strong enough to withstand, for example, thermal stress, such as that from boiling water. For the purposes of encapsulation of vitamins, pharmaceuticals, food additives, flavors, fragrances, photographic additives, etc. in proteins, it is desirable to crosslink the polymer to a degree that it is insoluble when placed in boiling water for at least 3 minutes. This level of gelatin crosslinking has surprisingly been found to be achievable with a sugar and a GRAS salt upon heating to relatively low temperatures (e.g. 55°C to 85°C) at which certain heat-sensitive ingredients (e.g. vitamin A) are not substantially degraded.

The preferred salt for use in the crosslinking process and product of the present invention is a salt which is categorized as "generally recognized as safe" (i.e. GRAS). GRAS has been defined by the United States Food and Drug Administration in parts 182, 184, and 582 of 21 Code of Federal Regulations (21 CFR).

The process of the present invention involves making a composition which comprises gelatin, among other ingredients, followed by heating the composition in order to crosslink the protein. The term "composition", as used herein, is meant to require that the combined ingredients are mixed to a degree of substantial uniformity. The composition can be a solution, an emulsion, or a gel. Preferably the composition is a gelled emulsion.

The composition comprises gelatin, a sugar, a salt, water, and preferably an additional ingredient which is to be en- capsulated or entrapped within a crosslinked gelatin matrix. However, while the gelatin may, in general have a bloom value of from 0 to 300, the gelatin preferably has a bloom value of from 50 to 300. It is believed that whereas vitamin supplements used in animal feeds typically utilize gelatin having a bloom value of 85 (i.e. from 80 bloom to 90 bloom), food additives usually utilize gelatin having a bloom value of from 200 to 300 and pharmaceuticals utilize all bloom values. Furthermore, the gelatin may be either a Type A or a Type B gelatin. Type A gelatin is obtained from acid processing of collagen. Type B gelatin is obtained from alkaline processing of collagen.

The composition is made by dissolving gelatin, the sugar, and the salt (and possibly the additional ingredient, depending upon its solubility in water) in water, and heating it to 60°C in order to completely dissolve the gelatin.

In general, the gelatin is present in the composition in an amount of from 10 weight percent to 70 weight percent, based on the weight of the composition. still more preferably the gelatin is present in the composition in an amount of from 10 weight percent to 30 weight percent, based on the weight of the composition. Most preferably the gelatin is present in the composition in an amount of 17 weight percent, based on the weight of the composition.

Preferably, the gelatin has a bloom of from 50 to 300. Most preferably the gelatin has a bloom value of 85 if the product of the process is to be utilized as a vitamin supplement for animal feed.

The composition further comprises a sugar being at least one member selected from the group consisting of fructose, and glucose. The term fructose is meant to include not simply pure fructose, but also high fructose corn syrup, isomers of fructose, as well as fructose- bearing mixtures such as invert sugar (a mixture of fructose and glucose). The term glucose is meant to include not simply pure glucose, but also isomers of glucose (such as mannose) as well as glucose-bearing mixtures such as high glucose corn syrup. Most preferably the sugar is high fructose corn syrup.

In general, the sugar is present in the composition in an amount of from 3 to 30, preferably from 5 to 20, most preferably 10, weight percent, based on the weight of the composition.

The composition further comprises a water-soluble salt of a carboxylic acid as well as other water soluble salts. More specifically, the water-soluble salt is at least one member selected from the group consisting of calcium sulfate, calcium phosphate, aluminum subacetate, sodium tartrate, and sodium glutarate and preferably of sodium acetate, calcium acetate, sodium propionate, calcium propionate, sodium benzoate, sodium carbonate, potassium carbonate.

In general, the salt is present in the composition in an amount of from 0.5 to 25, preferably from 1 to 10 and most preferably 7, weight percent, based on the weight of the composition.

The composition further comprises water. The water content of the composition is considerably greater before the heating step than after the heating step, because the initial water content of the composition must be high enough to dissolve the protein (gelatin), sugar, salt, and possibly even the additional ingredient(s) (if they are water soluble). However, once the ingredients are dissolved, the composition is most preferably allowed to gel, and thereafter the gel is preferably dried to a moisture content of from 15 weight percent to 3 weight percent, before the heating step is initiated. Most preferably, the moisture content is 7 weight percent.

Upon first making the composition, the water is present in an amount which is great enough to at least dissolve all of the gelatin, sugar, and salt present in the composition. In general, the water (i.e. moisture) content of the composition is from 3 to 90, preferably from 25 to 60, most preferably 48, weight percent, based on the weight of the composition.

After the gelatin, sugar, and salt are dissolved, during the heating step the moisture content may be reduced to a level down to as low as 3 weight percent, based on the total (reduced) weight of the composition. It has been found that the crosslinking reaction can be carried out at relatively low temperatures so long as the moisture content of the composition being heated is at least 3 weight percent. Most preferably the moisture content of the composition is 7 weight percent during the heating step.

The composition may further comprise an additional ingredient. Of course, the additional ingredient should be compatible with the composition. The phrase "compatible with the composition" means that the additional ingredient does not undergo some undesirable reaction with the salt, sugar, protein, etc. As examples of "incompatibility", the additional ingredient should not modify the gelatin so that the gelatin will not thereafter undergo crosslinking so that a water insoluble protein matrix cannot be formed. The additional ingredient should not be so volatile that it completely boils off of the composition at the temperatures required to keep the gelatin dissolved (e.g. 60°C), and the additional ingredient should not react with the gelatin, salt, or sugar so that any one of them are rendered insoluble under the conditions for carrying out the process of the present invention.

Examples of additional ingredients which may be used in the present invention include: vitamins, pharmaceuticals, flavors, fragrances, food additives, photographic additives, etc. There are thousands of possible additional ingredients for use in the present invention. The additional ingredient may be either encapsulated within the crosslinked gelatin matrix, or may be simply entrapped within the crosslinked gelatin matrix. If the additional ingredient is insoluble in the aqueous composition, and an emulsion is formed before the heating step, the result will be an additional ingredient which is encapsulated within the crosslinked protein matrix. If the additional ingredient is soluble in the aqueous composition, the result will be an additional ingredient which is entrapped within the crosslinked gelatin matrix.

Preferred additional ingredients are vitamins. still more preferred additional ingredients are fat-soluble vitamins, which are of course not substantially water soluble. The fat-soluble vitamins, when combined with the aqueous composition, are preferably thereafter agitated so that an emulsion is formed.

A most preferred additional ingredient is vitamin A oil. Since vitamin A oil is substantially insoluble in the aqueous composition, emulsification can be used to produce an encapsulated vitamin A product in which a crosslinked gelatin matrix is insoluble when placed in boiling water for at least three minutes. This is a very desirable result since the encapsulated vitamin A product can be utilized in feed formulations which are subjected to the harsh conditions (high temperature, high moisture, high pressure, and high shear) found in pelleting and extrusion processes. Just as importantly, the present invention enables this relatively high degree of crosslinking without subjecting the vitamin A oil to a temperature at which substantial degradation of the vitamin occurs. Vitamin A oil is particularly sensitive to heat, the vitamin A degrading substantially when heated at temperatures of 90°C for even relatively short periods of time (ego 4 hours). Heating the vitamin A oil to 120°C for a period of even just 12 minutes results in significant degradation of the vitamin. Even relatively small amounts of degradation of the vitamin A oil (e.g. 5% degradation) result in significant loss of value, since the cost of the vitamin A oil is so much greater than the cost of the other ingredients (i.e. the gelatin, sugar, and salt) utilized in the formulation.

In general, the additional ingredient may be present in the composition in an amount of from about 0.1 weight percent to 60 weight percent, based on the weight of the composition before the heating (or drying) step. Preferably the additional ingredient is present in an amount of from 5 weight percent to 55 weight percent. Most preferably the additional ingredient is present in an amount of from 22 weight percent.

Once the composition is made by combining the protein, sugar, salt, water, and additional ingredient, the composition is then heated in order to crosslink the gelatin. The heating step is carried out in order to crosslink the gelatin to a degree at which it is insoluble upon being placed in boiling water (i.e. at 100°C) for at least 3 minutes. Still more preferably the gelatin is crosslinked to a degree that it is substantially insoluble in boiling water for at least 15 minutes.

Throughout that period of the heating step during which the crosslinking reaction is taking place, it has been found necessary to keep the moisture content of the composition at a level of at least 3 weight percent, based on the total weight of the composition. In general, during the heating step the moisture content of the composition may be within the range of from 3 weight percent up to 90 weight percent. However, it has been found that the maximum amount of water which can be present during the crosslinking reaction varies depending upon the particular salt utilized. It has been found that if sodium carbonate is utilized as the salt, the water content may be at least as high as 60 weight percent, based on the total weight of the composition (see Example 11, infra). However, if the salt utilized is sodium acetate, the maximum amount of of water which can be present during the crosslinking reaction is 30 weight percent water.

For most of the salts which can be used in the present invention, the maximum amount of moisture at which the crosslinking reaction will occur is 30 weight percent.

The temperature range to be utilized for the crosslinking may be from 55°C to 180°C. However, if a heat-sensitive additional ingredient is present during the crosslinking step (i.e. an ingredient such as vitamin A, which begins to degrade at appreciable rates at temperatures around 90°C), it is preferable to carry out the heating step within a temperature range of from 55°C to 85°C. Most preferably the heating step is carried out at a temperature of 75°C.

The duration of the heating step is quite broad, depending upon the temperature employed in the process. If a relatively high temperature is employed (e.g. around 180°C), the heating step need be no longer than 30 seconds to a few minutes in order to produce the desired degree of crosslinking. However, if a high temperature is used (i.e. a temperature of at least 100°C), the composition is preferably a gel having a moisture content of less than 10 percent, by weight. If a relatively low temperature is employed (e.g. from around 55°C to around 80°C) the heating step may be carried out for a period of several hours (e.g. from 2 hours to 24 hours) in order to produce the desired degree of crosslinking.

A moisture content of at least 3 weight percent has been found to be necessary in order to sustain the crosslinking reaction. Thus,it is necessary to maintain this moisture level during that portion of the heating step that the crosslinking reaction is to progress. Further heating after the moisture content has dropped below 3 weight percent will not sustain further crosslinking of the protein, and is also undesirable if a heat-sensitive ingredient is present in the composition. As a general rule, the heating step should be carried out at a temperature below that at which any heat-sensitive ingredient degrades, and as a general rule the heating step should be no longer than that period required to produce the desired degree of crosslinking.

The present invention also relates to a crosslinked gelatin product which encapsulates (or entraps) an additional ingredient. In general, the product of the present invention comprises:
A. gelatin which is crosslinked to a degree at which it is substantially insoluble upon being placed in boiling water for at least 3 minutes;
B. a sugar wherein the sugar is at least one member selected from the group consisting of fructose and glucose;
C. a salt wherein the salt is at least one member selected from the group consisting of water-soluble salts of carboxylic acids, sodium carbonate, potassium carbonate, calcium sulfate, and calcium phosphate;
D. water; and
E. an additional ingredient, preferably vitamin A.

The crosslinked gelatin in the product of the present invention is as described above.

In general, the crosslinked protein is present in the product in an amount of from 10 weight percent to 70 weight percent, based on the weight of the product. Preferably the crosslinked protein is present in an amount of from 15 weight percent to 50 weight percent, based on the weight of the product. Most preferably the protein is present in an amount of about 30 weight percent, based on the weight of the product.

In general, the sugar is present in the product in an amount of from 3 weight percent to 30 weight percent, based on the weight of the product. Preferably the sugar is present in the product in an amount of from 10 weight percent to 30 weight percent, based on the weight of the product. Most preferably the sugar is present in an amount of 20 weight percent, based on the weight of the product.

The product further comprises a water-soluble salt as defined above, most preferably sodium acetate.

The water soluble salt is present in the product in an amount of from 0.5 to 25, preferably from 2 to 10 and most preferably 5, weight percent, based on the weight of the product.

The product further comprises water in an amount of from 1 to 18, preferably from 3 to 13 and most preferably of 4, weight percent, based on the weight of the product. If a vitamin particulate is made according to a most preferred embodiment of the invention (see Example 3, infra), it is most preferred that the product is dried to a moisture content of 4 weight percent, based on the weight of the product.

The product may further comprise an additional ingredient. Examples of additional ingredients which may be used in the present invention include: vitamins, pharmaceuticals, flavors, fragrances, food additives, photographic additives, etc. There are thousands of possible additional ingredients for use in the present invention. The additional ingredient may be either encapsulated within the crosslinked protein matrix, or may be simply entrapped within the crosslinked protein matrix. If the additional ingredient is insoluble in water, the product will comprise an additional ingredient which is encapsulated within the crosslinked gelatin matrix.

The additional ingredient may be a water-soluble vitamin. The water-soluble vitamin will be entrapped within the crosslinked protein matrix. The water-soluble vitamin may be at least one member selected from the group consisting of vitamin C, thiamine, pyridoxine, riboflavin, biotin, nicotinamide, folic acid, cobalamin, and pantothenic acid.

More preferably the additional ingredient is a fat-soluble vitamin which is at least one member selected from the group consisting of vitamin A, carotinoids, vitamin D, vitamin E, and vitamin K. The fat-soluble vitamin is encapsulated within the crosslinked protein (preferably gelatin) matrix. The most preferred additional ingredient is vitamin A oil.

Small quantities of other ingredients including antioxidants, butylated hydroxy anisole (BHA), butylated hydroxy toluene (BHT), ethoxyquin (6-ethoxy-1, 2-dihydro-2,2, 4-trimethyl-quinoline), and the like; and humectants, such as glycerin, sorbitol, polyethylene glycol, and the like; emulsifiers, such as lecithin; extenders and solubilizers; coloring agents; and complexing agents; can also be incorporated into the composition made in the process and product of the present invention.

In general, the additional ingredient may be present in the product in an amount of from 0.1 to 60, preferably 10 to 50, most preferably of 40, weight percent, based on the weight of the product.

### EXAMPLES

### EXAMPLE 1: Preparation of Gelatin Slabs

The gelatin compositions described in the examples below were prepared by dissolving gelatin and other ingredients in water at 60°C, then allowing the resulting solutions to set, or gel, at ambient temperature, into slabs approximately 1 to 2 millimeters in thickness and approximately 75 millimeters in diameter. The gelled slabs were then allowed to dry at ambient temperature and humidity for about 16-20 hours, with a final moisture content of 25 weight percent (based on the weight of the slab).

### EXAMPLE 2

Three solutions (solutions A, B and C) were prepared. Each solution contained 18.6 parts by weight Type B gelatin having a bloom value of from 80 to 90 dissolved in 50.2 parts (by weight) water heated at 60°C to dissolve the gelatin. Additionally, solution A contained fructose (3 parts), solution B contained calcium acetate (3 parts), and solution C contained calcium acetate (3 parts) and fructose (3 parts).

Two slabs were prepared from each solution, via the procedure described in Example 1, supra. One slab from each solution was placed in an oven at 70°C for 6 hours, then cooled to ambient temperature. The other slabs were not heated, these slabs being used as controls. Upon completion of the heating, all six slabs were then placed in boiling water with stirring. The control slabs for solutions A, B and C, as well as the heat-treated slabs for solutions A and B underwent substantially complete dissolution in less than 3 minutes, indicating an absence of any substantial amount of crosslinking. The heat-treated slab from solution C remained substantially insoluble after 10 minutes, indicating a substantial degree of crosslinking.

This example shows the need for both sugar and salt in order to effectuate substantial crosslinking.

### EXAMPLE 3

High fructose corn syrup (13.4 parts) and sodium acetate (2.1 parts) were dissolved in water (43 parts). Gelatin (19.2 parts, 80-90 Bloom, Type B) was added, and the solution was heated to 60°C to dissolve the gelatin. Vitamin A acetate oil (22.3 parts of oil having 2.1 million international units (MIU) per gram) containing ethoxyquin (80 mg/MIU vitamin A) and BHT (10 mg/MIU vitamin A) was added and the resulting mixture was homogenized at 60°C, resulting in an aqueous emulsion with oil droplets approximately 2 microns in diameter.

The emulsion was then spray-congealed using hydrophobic starch as the absorbant. The vitamin-active beadlets were then separated from the excess starch so that a product was obtained in which the beadlets ranged in size between about 105 microns to about 840 microns. The resulting beadlets were dried in a fluid-bed dryer to a moisture of 6.0 weight percent. The beadlets were then heated to 75°C for eight hours in the fluid-bed dryer with humidified air so that the moisture of the beadlets was maintained between 6 and 9 weight percent. When the heating was complete, the beadlets were dried to a final moisture content of 4.1%. The final product was substantially crosslinked, being substantially insoluble in boiling water for longer than 15 minutes.

### EXAMPLE 4

Three gelatin solutions were prepared by dissolving gelatin (18.6 parts, 80-90 Bloom Type B), fructose (5.3 parts) and calcium acetate (5.0 parts) in water (50.2 parts) heated at 60°C. Calcium hydroxide was added to each solution (0.15, 0.30 and 0.45 parts, respectively) to adjust the pH (at 60°C) of the solutions to 7.0, 8.0 and 9.0, respectively.

A slab was prepared for each solution as described in Example 1 (supra). The slabs were then heated at 70°C for 6 hours. During heating, all three slabs turned color from straw-colored to dark brown. The slabs were then placed in boiling water with stirring. All three slabs were substantially insoluble after 12 minutes, indicating each was slab substantially crosslinked. However, the gelatin matrix integrity appeared to increase with increasing pH.

This example illustrates the effect of pH on the process for producing a crosslinked gelatin matrix.

### EXAMPLE 5

Fructose (3.0 parts), sodium acetate (5.0 parts) and hydrolyzed gelatin (18.6 parts) were dissolved in water (50.2 parts) heated at 60°C. Two slabs were prepared as described in Example 1, supra. One slab was placed in an oven at 70°C for 6 hours, then cooled to ambient temperature, the other slab was kept as a control. The heated slab turned from straw-colored to dark brown during heating. Both slabs were then placed in boiling water with stirring. The control slab underwent substantially complete dissolution in less than 1 minute, while the heat-treated slab took longer (less than 3 minutes) to completely dissolve.

These results indicate that hydrolyzed gelatin can be crosslinked by the process of the present invention, but not to the same degree as unhydrolyzed gelatin.

### EXAMPLE 6

Fructose (5.2 parts), calcium acetate (4.3 parts), glycerin (2.2 parts) and caramel color (2.4 parts) were dissolved in water (50.3 parts). Gelatin (22.9 parts, 80-90 Bloom Type B) was added and the solution was heated to 60°C to dissolve the gelatin. Vitamin A acetate oil (24.3 parts of 2.1 MIU/g) containing ethoxyquin (80 mg/MIU vitamin A) and BHT (10 mg/MIU vitamin A) was added, and the resulting mixture was homogenized at 60°C, resulting in an aqueous emulsion with oil droplets approximately 2 microns in diameter.

The emulsion was then spray-congealed using hydrophobic starch as the absorbant. The vitamin-active beadlets were then separated from the excess starch so that a product was obtained in which the beadlets ranged in size between 105 microns to 840 microns. The resulting product was dried in a fluid-bed dryer to a moisture of 8.0 weight percent. The product was then heated in the fluid-bed to 75°C for 8 hours with hot, humidified air, so that the moisture content of the product was maintained between 6 and 9 weight percent during the course of heating. When the heating was complete, the product was dried to a final moisture content of 4.1%. The final product was substantially crosslinked, being substantially insoluble in boiling water for greater than 15 minutes.

### EXAMPLE 7

Three solutions, A, B, and C, were prepared, each containing gelatin (18.6 parts, 80-90 Bloom, Type B) dissolved in water (50.2 parts) heated at 60o C. Additionally, solution A contained sodium acetate (5 parts) and sucrose (3 parts), solution B contained calcium acetate (5 parts) and sucrose (3 parts), and solution C contained sucrose (3 parts), but no salt. Slabs were prepared for each solution as described in Example 1 (supra), then heated at 70°C for 6 hours. No color change was observed during heating for any of the slabs. After cooling to ambient temperature, the slabs were placed in boiling water with stirring. All three slabs underwent substantially complete dissolution in less than 2 minutes, indicating that no substantial crosslinking occurred during heating of any of the slabs.

### EXAMPLE 8

Five solutions were prepared containing sodium acetate (5 parts), gelatin (18.6 parts, 80-90 Bloom, Type B) and water (50.2 parts). Each solution was heated to 60°C in order to dissolve the gelatin. In addition, each solution contained one of the following sugars: glucose (5.6 parts), mannose (5.6 parts), invert sugar (5.6 parts), corn syrup (4 parts, containing approximately 75 weight percent solids) and high fructose corn syrup (5.6 parts). Slabs were prepared for each solution as described in Example 1 (supra). The five slabs were then heated at 70°C for 6 hours. The glucose, mannose, invert sugar and high fructose corn syrup slabs all turned dark brown during heating. The corn syrup slab turned amber during heating.

After heating, the slabs were allowed to cool to ambient temperature. The cooled slabs were then placed in boiling water, with stirring. All 5 slabs remained substantially insoluble after at least 5 minutes in boiling water, indicating they were all substantially crosslinked.

### EXAMPLE 9

Three solutions, A, B and C, were prepared by dissolving gelatin (11.4 parts, 80-90 Bloom, Type A) in 88.6 parts water heated to 45-50°C. Additionally, solutions A and B both contained aluminum subacetate filtrate (ASF) solution (6.58 parts stock solution diluted with 13.16 parts water, prepared fresh as described by Shank in U.s. Pat. No. 4,500,453, column 8, lines 1- 67) which was added slowly with vigorous stirring, being careful to maintain the temperature of the gelatin solutions above 35°C. Solution B also contained fructose (2.7 parts). In addition to gelatin and water, solution C also contained undiluted ASF stock solution (18 parts) and fructose (2.7 parts).

Once addition of the ASF solution was complete, two slabs were prepared for each solution as described in Example 1, supra. Then, one slab for each solution was heated at 70°C for 6 hours, while the other slab was kept as a control. All 3 control slabs were clear and colorless.

After heating, the appearance of slab A remained unchanged, but slabs B and C had darkened. All 6 slabs were then placed in boiling water with stirring. The 3 control slabs underwent substantially complete dissolution in less than 2 minutes. Heat-treated slabs A and B underwent substantially complete dissolution in less than 3 minutes. Heat-treated slab C completely dissolved in less than 4 minutes.

### EXAMPLE 10

Four solutions (A, B, C and D) were prepared, each containing gelatin (18.6 parts, 80-90 Bloom, Type B) and fructose (3 parts) dissolved in water (50.2 parts) which was heated to 60°C. Additionally, solution A contained sodium propionate (3 parts) and glycerin (2.3 parts), solution B contained calcium propionate (3 parts) and glycerin (2.3 parts), solution C contained sodium benzoate (5.0 parts) and solution D contained potassium carbonate (5.0 parts). slabs were prepared for each solution as described in Example 1, supra and heated at 70°C for 6 hours. All four slabs turned dark brown during heating. The slabs were then placed into boiling water with stirring. All four slabs remained substantially insoluble after 15 minutes in boiling water, indicating each slab underwent a substantial degree of crosslinking.

### EXAMPLE 11

Gelatin (18.6 parts, 80-90 Bloom Type B), fructose (3.0 parts) and sodium carbonate (5.0 parts) were dissolved in water (50.2 parts) heated at 60°C. Once the gelatin had dissolved, the temperature of the solution was increased to 75°C, at which point a strong amine odor developed, followed by a rapid increase in viscosity. Within 10 minutes at 75°C the solution had set into a gel which was dark amber in color and insoluble in water.

This Example illustrates that the process of the present invention can be carried out using water in an amount of 60 weight percent, based on the weight of the composition. However, further experiments have revealed that the process can be carried out using water in an amount of 80 weight percent. Therefore, it is believed that the process can be carried out with a composition comprising water in an amount of as high as 90 weight percent, based on the weight of the composition.

## Claims

1. A method comprising the steps of:
A. making a composition which is an aqueous solution, wherein the solution comprises:
i. 10 to 70 weight percent of a gelatin;
ii. 3 to 30 weight percent of a sugar, wherein the sugar is at least one member selected from the group consisting of fructose and glucose;
iii. 0.5 to 25 weight percent of a salt, wherein the salt is at least one member selected from the group consisting of sodium acetate, calcium acetate, sodium propionate, calcium propionate, sodium benzoate, sodium carbonate, potassium carbonate, calcium sulfate, calcium phosphate, aluminum subacetate, sodium tartrate, and sodium glutarate;
iv. water in an amount of from 3 to 86 weight percent;
v. 0.1 to 60 weight percent of an additional ingredient which can be emulsified in an aqueous system; and
B. agitating the solution so that an emulsion is formed;
C, reducing the water content of the emulsion until the emulsion has a moisture content of from about 15 weight percent to 4 weight percent;
D. heating the composition to a temperature range of from 55°C to 180°C while maintaining the moisture content of the composition at a level of at least 3 weight percent, all percentages being based upon the weight of the composition,
so that the gelatin is crosslinked to a degree at which it is water insoluble upon being placed in water at 100°C for at least 3 minutes.

2. A process as described in claim 1 wherein the additional ingredient is vitamin A oil.

3. A process as described in claim 1 or 2, wherein
A. the gelatin has a bloom of from 50 to 300, the gelatin being present in an amount of from about 10 weight percent to 30 weight percent, based upon the weight of the composition;
B. the sugar is present in an amount of from 5 weight percent to 20 weight percent, based upon the weight of the composition;
C. the salt is sodium acetate and is present in an amount of from 1 weight percent to 10 weight per- cent, based upon the weight of the composition; and
D. the additional ingredient is a fat-soluble vitamin and is present in an amount of from 5 weight percent to 55 weight percent.

4. A crosslinked gelatin product obtained by the process of anyone of claims 1 - 3, comprising:
A. 10 to 70 weight percent of a gelatin which is crosslinked to a degree at which it is water insoluble upon being placed in water at 100°C for at least 3 minutes, having a bloom of from 50 to 300, the gelatin being present in an amount of from 10 weight percent to 70 weight percent;
B. 3 to 30 weight percent of a sugar, wherein the sugar is at least one member selected from the group consisting of fructose and glucose;
C. 2 to 10 weight percent of a salt, wherein the salt is at least one member selected from the group consisting of sodium acetate, calcium acetate, sodium propionate, calcium propionate, sodium benzoate, sodium carbonate, potassium carbonate, calcium sulfate, calcium phosphate, aluminum subacetate, sodium tartrate, and sodium glutarate;
D. 3 to 13 weight percent of water;
E. 0 to 60 weight percent of an additional ingredient selected from the group consisting of vitamins, pharmaceuticals, food additives, flavors, fragrances, and photographic additives, all percentages being based upon the weight of the product.

5. A product as claimed in claim 4 wherein the salt is sodium acetate and the additional ingredient is vitamin A.

## Patentansprüche

1. Verfahren, dadurch gekennzeichnet, daß man:
A. eine Zusammensetzung in Form einer wäßrigen Lösung herstellt, enthaltend:
i. 10 bis 70 Gewichtsprozent einer Gelatine,
ii. 3 bis 30 Gewichtsprozent eines Zuckers, wobei es sich bei dem Zucker um mindestens einen aus der Gruppe bestehend aus Fructose und Glucose handelt,
iii. 0,5 bis 25 Gewichtsprozent eines Salzes, wobei es sich bei dem Salz um mindestens eines aus der Gruppe bestehend aus Natriumacetat, Calciumacetat, Natriumpropionat, Calciumpropionat, Natriumbenzoat, Natriumcarbonat, Kaliumcarbonat, Calciumsulfat, Calciumphosphat, basischem Aluminiumacetat, Natriumtartrat und Natriumglutarat handelt,
iv. Wasser in einer Menge von 3 bis 86 Gewichtsprozent,
v. 0,1 bis 60 Gewichtsprozent eines zusätzlichen Bestandteils, der sich in einem wäßrigen System emulgieren läßt, und
B. die Lösung so rührt, daß eine Emulsion entsteht,
C. den Wassergehalt der Emulsion bis zu einem Feuchtigkeitsgehalt der Emulsion von etwa 15 Gewichtsprozent bis 4 Gewichtsprozent verringert,
D, die Zusammensetzung auf eine Temperatur im Bereich von 55°C bis 180°C erhitzt und dabei den Feuchtigkeitsgehalt der Zusammensetzung bei mindestens 3 Gewichtsprozent hält, wobei sich alle Prozentangaben auf das Gewicht der Zusammensetzung beziehen,
so daß die Gelatine dabei in einem solchen Ausmaß vernetzt wird, daß sie nach Einbringen in Wasser von 100°C mindestens 3 Minuten lang wasserunlöslich ist.

2. Verfahren nach Anspruch 1, dadurch gekennzeichnet, daß man als zusätzlichen Bestandteil Vitamin-A-Öl einsetzt.

3. Verfahren nach Anspruch 1 oder 2, dadurch gekennzeichnet, daß man
A. eine Gelatine mit einem Bloom-Wert von 50 bis 300 in einer Menge von etwa 10 Gewichtsprozent bis 30 Gewichtsprozent, bezogen auf das Gewicht der Zusammensetzung,
B. den Zucker in einer Menge von 5 Gewichtsprozent bis 20 Gewichtsprozent, bezogen auf das Gewicht der Zusammensetzung,
C. als Salz Natriumacetat in einer Menge von 1 Gewichtsprozent bis 10 Gewichtsprozent, bezogen auf das Gewicht der Zusammensetzung, und
D. als zusätzlichen Bestandteil ein fettlösliches Vitamin in einer Menge von 5 Gewichtsprozent bis 55 Gewichtsprozent einsetzt.

4. Vernetztes Gelatineprodukt gemäß einem Verfahren nach einem der Ansprüche 1 - 3, enthaltend:
A. 10 bis 70 Gewichtsprozent einer Gelatine, die in einem solchen Ausmaß vernetzt ist, daß sie nach Einbringen in Wasser von 100°C mindestens 3 Minuten lang wasserunlöslich ist und einen Bloom-Wert von 50 bis 300 aufweist, wobei die Gelatine in einer Menge von 10 Gewichtsprozent bis 70 Gewichtsprozent vorliegt,
B. 3 bis 30 Gewichtsprozent eines Zuckers, wobei es sich bei dem Zucker um mindestens einen aus der Gruppe bestehend aus Fructose und Glucose handelt,
C. 2 bis 10 Gewichtsprozent eines Salzes, wobei es sich bei dem Salz um mindestens eines aus der Gruppe bestehend aus Natriumacetat, Calciumacetat, Natriumpropionat, Calciumpropionat, Natriumbenzoat, Natriumcarbonat, Kaliumcarbonat, Calciumsulfat, Calciumphosphat, basischem Aluminiumacetat, Natriumtartrat und Natriumglutarat handelt,
D. 3 bis 13 Gewichtsprozent Wasser,
E. 0 bis 60 Gewichtsprozent eines zusätzlichen Bestandteils aus der Gruppe der Vitamine, Pharmazeutika, Lebensmittelzusätze, Geschmacksstoffe, Geruchsstoffe und fotografischen Zusatzstoffe, wobei sich alle Prozentangaben auf das Gewicht des Produkts beziehen.

5. Produkt nach Anspruch 4, dadurch gekennzeichnet, daß es als Salz Natriumacetat und als zusätzlichen Bestandteil Vitamin A enthält.

## Revendications

1. Un procédé comprenant les étapes suivantes :
A. préparer une composition qui est une solution aqueuse, la solution comprenant :
i. 10 à 70 pour cent en poids d'une gélatine ;
ii. 3 à 30 pour cent en poids d'un sucre, le sucre étant au moins un membre du groupe formé par le fructose et le glucose ;
iii. 0,5 à 25 pour cent en poids d'un sel, le sel étant au moins un membre du groupe formé par l'acétate de sodium, l'acétate de calcium, le propionate de sodium, le propionate de calcium, le benzoate de sodium, le carbonate de sodium, le carbonate de potassium, le sulfate de calcium, le phosphate de calcium, le subacétate d'aluminium, le tartrate de sodium et le glutarate de sodium ;
iv. de l'eau en une quantité de 3 à 86 pour cent en poids ;
v. 0,1 à 60 pour cent en poids d'un ingrédient supplémentaire qui peut être émulsionné dans un système aqueux ; et
B. agiter la solution pour former une émulsion ;
C. réduire la teneur en eau de l'émulsion jusqu'à ce que l'émulsion ait une teneur en humidité d'environ 15 pour cent en poids à 4 pour cent en poids ;
D. chauffer la composition dans un intervalle de température de 55°C à 180°C tout en maintenant la teneur en humidité de la composition à une valeur d'au moins 3 pour cent en poids, tous les pourcentages étant exprimés par rapport au poids de la composition,
de sorte que la gélatine soit réticulée à un degré auquel elle est insoluble dans l'eau en étant placée dans l'eau à 100°C pendant au moins 3 minutes.

2. Un procédé tel que décrit dans la revendication 1, dans lequel l'ingrédient supplémentaire est une huile à la vitamine A.

3. Un procédé tel que décrit dans la revendication 1 ou 2, dans lequel
A. la gélatine a un degré Bloom de 50 à 300, la gélatine étant présente en une quantité d'environ 10 pour cent en poids à 30 pour cent en poids, par rapport au poids de la composition ;
B. le sucre est présent en une quantité de 5 pour cent en poids à 20 pour cent en poids, par rapport au poids de la composition ;
C. le sel est l'acétate de sodium et il est présent en une quantité de 1 pour cent en poids à 10 pour cent en poids, par rapport au poids de la composition ; et
D. l'ingrédient supplémentaire est une vitamine liposoluble et il est présent en une quantité de 5 pour cent en poids à 55 pour cent en poids.

4. Un produit à base de gélatine réticulée obtenu par le procédé de l'une quelconque des revendications 1 à 3, comprenant :
A. 10 à 70 pour cent en poids d'une gélatine qui est réticulée jusqu'à un degré auquel elle est insoluble dans l'eau en étant placée dans l'eau à 100°C pendant au moins 3 minutes, ayant un degré Bloom de 50 à 300, la gélatine étant présente en une quantité de 10 pour cent en poids à 70 pour cent en poids ;
B. 3 à 30 pour cent en poids d'un sucre, le sucre étant au moins un membre du groupe formé par le fructose et le glucose ;
C. 2 à 10 pour cent en poids d'un sel, le sel étant au moins un membre du groupe formé par l'acétate de sodium, l'acétate de calcium, le propionate de sodium, le propionate de calcium, le benzoate de sodium, le carbonate de sodium, le carbonate de potassium, le sulfate de calcium, le phosphate de calcium, le subacétate d'aluminium, le tartrate de sodium et le glutarate de sodium ;
D. 3 à 13 pour cent en poids d'eau ;
E. 0 à 60 pour cent en poids d'un ingrédient supplémentaire choisi dans le groupe formé par les vitamines, les agents pharmaceutiques, les additifs alimentaires, les aromatisants, les parfums et les additifs photographiques, tous les pourcentages étant exprimés par rapport au poids du produit.

5. Un produit tel que revendiqué dans la revendication 4, dans lequel le sel est l'acétate de sodium et l'ingrédient supplémentaire est la vitamine A.
